# FASCICULE DE BREVET EUROPEEN

(11) **EP 3 661 754 B1**
(45) Date de publication et mention de la délivrance du brevet: **07.07.2021**
(21) Numéro de dépôt: 18749799.5
(22) Date de dépôt: 03.08.2018
(51) Int. Cl.: B41J 2/175, B29C 64/106, B29C 64/209, B33Y 10/00, B33Y 30/00, C12M 1/26

(54) **TÊTE D'IMPRESSION D'UNE IMPRIMANTE, IMPRIMANTE ET PROCÉDÉ D'IMPRESSION**
DRUCKKOPF EINES DRUCKERS, DRUCKER UND DRUCKVERFAHREN
PRINT HEAD OF A PRINTER, PRINTER AND PRINTING METHOD

(30) Priorité: 03.08.2017 FR 1757496
(43) Date de publication de la demande: 10.06.2020
(73) Titulaire: Centre National de la Recherche Scientifique, 75016 Paris (FR); Institut Curie, 75248 Paris Cedex 05 (FR); Sorbonne Université, 75006 Paris 6 (FR)
(72) Inventeur: MALAQUIN, Laurent, 31450 Ayguesvives (FR); VIOVY, Jean Louis, 75013 Paris (FR); SOULEILLE, Sandrine, 31750 Escalquens (FR); DOLLAT, Xavier, 31120 Roques sur Garonne (FR); FOURNIE, Victor, 31670 Labège (FR)
(74) Mandataire: Gevers & Orès
(86) Numéro de dépôt international: PCT/EP2018/071189
(87) Numéro de publication internationale: WO 2019/025615

(56) Documents cités:
- WO-A1-2014/197999
- US-A1- 2015 037 445
- US-A1- 2017 210 069
- SANGJUN MOON ET AL: "Layer by Layer Three-dimensional Tissue Epitaxy by Cell-Laden Hydrogel Droplets", TISSUE ENGINEERING PART C: METHODS, vol. 16, no. 1, 1 février 2010 (2010-02-01), pages 157-166, XP055180383, ISSN: 1937-3384, DOI: 10.1089/ten.tec.2009.0179

## Description

L'invention a trait au domaine de la bioimpression et plus particulièrement aux imprimantes.

La bioimpression permet des avancées médicales considérables. Parmis ces avancées on pourra citer la réalisation d'implants ou de structures vivantes constituées de cellules et de matrice extracellulaire, comme par exemple de la peau ou encore des structures osseuses.

A l'heure actuelle, la bioimpression est principalement utilisée pour la recherche médicale. La bioimpression permet la réalisation de microenvironnements de culture cellulaires et de modèles de tissus vivants utiles au diagnostic et à l'analyse des mécanismes à l'origine de certaines pathologies. Les scientifiques l'utilisent notamment pour étudier l'évolution des cellules tumorales ou des cellules souches. Ceci leur permet de mieux comprendre l'évolution de ces cellules, cancéreuses notamment, pour mieux soigner et prémunir l'humanité des cancers.

Il est établi que les cellules cancéreuses, pour ne prendre que cet exemple, ont besoin d'un microenvironnement tridimensionnel spacialement hétérogène. Les composantes de ce microenvironnement sont physiques (topologie, propriétés mécaniques, etc...), chimiques (composition de la matrice extracellulaire, concentration de facteurs biochimiques, etc...) et (distribution spatiale des différents types cellulaires qui composent le tissu, etc...). En bioimpression, le microenvironnement tridimensionnel physiologique est reconstruit pour permettre de contrôler les processus de prolifération et de différenciation cellulaires tout en garantissant la fonctionnalité des tissus générés sur le long terme.

A terme, l'objectif visé par les scientifiques est la réalisation de modèles de tissus fonctionnels, biomimétriques, implantés. Il s'agit par exemple d'organes humains « bioimprimés » dans le cadre de la médecine régénérative. Il s'agit de modèles de tissus fonctionnels, mimant ou non une pathologie, qui peuvent être utilisé pour des applications pharmacologiques ou thérapeutiques.

Plusieurs technologies sont actuellement utilisées pour la bioimpression. Toutefois ces technologies présentent chacune des inconvénients particulièrement limitatifs pour les scientifiques.

Dans ce qui suit, on entend par « encre», un matériau photosensible, comportant un polymère ou un hydrogel ou un mélange de polymères ou d'hydrogels, comprenant ou pas des cellules vivantes et comportant ou pas des molécules additionnelles telles que des protéines, ADN, acides, bases, sucres, facteurs de croissance, peptides, marqueurs, particules chargées, molécules et colloïdes.

Une première technologie est une technique de micro-extrusion. Cette technique consiste à utiliser une ou plusieurs tête(s) d'impression. Chaque tête permet de déposer une fibre d'hydrogel générée par extrusion au travers d'une buse, cette fibre ayant un diamètre variable en fonction du diamètre de la buse. Les fibres sont successivement juxtaposées et superposées de façon à générer une structure en trois dimensions. Cet hydrogel est ensemencé ou pas avec des cellules. Dans le cas où il ne s'agit que d'un matériau seul, l'impression des structures qui servent de support de culture, est généralement suivie d'une immersion dans une suspension de cellules qui se déposent à sa surface. Les encres sont poussées à travers une micro-seringue et déposées à l'aide d'une aiguille. Cette technologie comprend de multiples inconvénients. La taille de l'aiguille détermine en premier lieu la résolution des structures obtenues. Les aiguilles ont un diamètre donné usuellement de l'ordre de la centaine de micromètres, et permettent donc l'impression de matériaux avec une résolution correspondante, de l'ordre de 100 µm. Par ailleurs, le contrôle de la résolution dans l'axe d'extrusion des fibres est difficile à contrôler et généralement supérieur à 100 µm, celle-ci n'étant pas déterminée par le diamètre de la buse mais par le contrôle des écoulements. Cette technologie se heurte à un problème d'envergure, puisque pour augmenter la résolution, les aiguilles aux diamètres les plus petits engendrent une augmentation des forces de cisaillement durant l'extrusion, provoquant la détérioration des propriétés des gels ou bien une perte de viabilité des cellules présentes éventuellement dans le matériau extrudé (dans l'encre). Les forces de cisaillement qui s'appliquent aux cellules au cours de leur trajet, peuvent être délétaires, c'est-à-dire entrainer une apoptose, notamment si le diamètre de l'aiguille est trop petit et/ou si la vitesse d'extrusion est trop importante. Par ailleurs, cette technologie implique des temps d'impression relativement longs. A noter aussi, que la réalisation de structures hétérogènes, c'est-à-dire nécessitant l'utilisation successives de différentes encres, s'avère être laborieuse. L'impression d'encres multiples, se traduit par l'utilisation séquentielle et l'alignement de plusieurs têtes d'extrusion contenant chacune des encres à imprimer. Il est impossible de mélanger ces matériaux lors de l'extrusion pour ajuster leurs concentrations relatives. Par ailleurs, la résolution limitée de cette approche (100 µm soit 10 fois la taille d'une cellule) ne permet pas de contrôler précisément l'organisation des structures imprimées.

Une deuxième technologie est une technique jet-d'encre. La technique jet d'encre consiste en une tête d'impression qui projette des micro-gouttelettes d'encre . Cette technique présente une résolution d'environ cinquante micromètres et nécessite de prendre des précautions importantes pour conserver la viabilité cellulaire lors de l'écriture. En effet, les dispositifs utilisés pour générer les microgouttes sont basés sur des effets thermiques ou piezo éléctrique qui induisent des contraintes de cisaillement, de compression et d'élévation de température importantes, qui peuvent nuire à la viabilité cellulaire.

Une troisième technologie est une technique d'impression par transfert assisté par laser. Cette technique consiste à disposer sur une lamelle métallisée une pellicule d'encre. Dans cette technique d'impression, un laser est dirigé à l'aide d'un miroir puis focalisé par une lentille et enfin frappe la lamelle du côté opposé à celui où est déposée l'encre . Cette technologie permet d'otenir une précision de l'ordre du micromètre. La technique d'impression assistée par laser présente des inconvénients. Parmi ces inconvénients, les constructions réalisées sont instables lorsqu'il s'agit de créer des structures en trois dimensions et tout particulièrement lorsqu'il s'agit de créer des structures creuses ou pleines avec de forts rapports d'aspects, c'est-à-dire avec des formes complexes. De plus, les structures hétérogènes sont particulièrement difficiles à réaliser du fait de la nécessité d'utiliser séquentiellement une lamelle différente pour chaque encre. Dans cette technique, chaque lamelle comprend un type de cellules. Lorsqu'une cellule est frappée par le laser, celle-ci est éjectée et se dépose sur un substrat. Pour ajouter une cellule différente et créer une structure hétérogène, il faut utiliser une autre lamelle sur laquelle est disposé un autre type de cellules, en s'assurant que la lamelle en question est parfaitement alignée. Ceci est donc fastidieux et requiert un niveau de précision difficile à soutenir car des manipulations (répétées) de lamelles sont nécessaires. Une structure hétérogène est une structure composée de cellules différentes, de matrices extracellulaires différentes et/ou de biomolécules différentes.

A noter en outre, que pour produire un tissu, il existe un besoin de reproduire l'hétérogénéité de ce tissu à l'échelle de la cellule individuelle. Cette hétérogénéité comprend, le type de cellule et sa position spatiale, la concentration des espèces et leur concentration spatiale, le type de matrice extracellulaire, sa porosité, ses propriétés physicochimiques, mécaniques, et la distribution spatiale de ces propriétés.

Une tête d'impression microfluidique est divulguée dans le document WO 2014/197999 A1.

L'invention vise à remédier à l'un au moins de ces inconvénients.

Un objectif de l'invention est de proposer une tête d'impression micro-fluidique d'une imprimante, qui puisse offrir la possibilité de créer des structures hétérogènes tout en ayant une résolution au moins inférieures à 100 micromètres, de préférence inférieure à 50 micromètres et plus préférablement inférieure à 20 micromètres.

Un autre objectif est de proposer une imprimante capable d'imprimer sur demande une structure composée d'un ou plusieurs type(s) d'encre(s).

Un autre objectif est de proposer une imprimante dans laquelle les flux d'encres peuvent être contrôlés en temps réel pour créer un mélange dont la composition en encres est minutieusement ajustée.

Un autre objectif est de proposer une imprimante dans laquelle les quantités d'encres utilisées sont minimisées.

Un autre objectif est de proposer une imprimante qui puisse imprimer dans les trois directions de l'espace et ceci avec une résolution au moins inférieure à 100 micromètres, de préférence inférieure à 50 micromètres et plus préférablement inférieure à 20 micromètres.

Un autre objectif est de proposer une imprimante qui puisse imprimer avec différentes longueurs d'ondes, incluant la lumière UV, visible et infra rouge.

Un autre objectif est de proposer une imprimante capable d'imprimer selon différentes résolutions.

Un autre objectif est de proposer une imprimante qui puisse imprimer avec différentes résolutions de façon à optimiser les vitesses d'impression.

Un autre objectif est de proposer une imprimante capable d'imprimer en utilisant plusieurs types d'encres.

Un autre objectif est de proposer une imprimante permettant de réaliser une impression en maintenant les structures immergées dans un milieu de culture tout au long du processus d'impression.

A cet effet, il est proposé en premier lieu une tête d'impression microfluidique d'une imprimante dans laquelle la tête comprend une face distale et :
- au moins un premier canal appelé canal d'injection pour injecter une encre sur un substrat, le canal d'injection débouchant sur la face distale par un premier orifice appelé orifice d'injection,
- au moins un deuxième canal appelé canal d'aspiration pour aspirer l'encre entre autres, le canal d'aspiration débouchant sur la face distale par un deuxième orifice appelé orifice d'aspiration,
- une gorge définie sur la face distale et dans laquelle l'orifice d'aspiration débouche,
- au moins une source de lumière ou un guide d'onde optique pour transmettre la lumière émise par une source de lumière,
et dans laquelle la source de lumière ou le guide d'onde optique est apte à projeter un faisceau lumineux sur une zone éclairante située sur la face distale, la zone éclairante étant à son tour apte à projeter un faisceau lumineux depuis la face distale.

Diverses caractéristiques supplémentaires peuvent être prévues seules ou en combinaison :
- la tête d'impression comprend un corps et un embout rapporté sur le corps, l'embout comprenant l'orifice d'injection, l'orifice d'aspiration, la gorge et la zone éclairante ;
- le corps comprend le canal d'injection, le canal d'aspiration et la source de lumière ou le guide d'onde optique, respectivement situés en regard de l'orifice d'injection, de l'orifice d'aspiration et de la zone éclairante ;
- l'embout est réalisé en matériau transparent, de préférence élastomère Poly Dimethil Siloxane ;
- la gorge s'étend en profondeur selon un axe longitudinal de l'embout et présente une forme entourant la zone éclairant ;
- la gorge s'étend sur la circonférence de l'embout **(24)** selon un secteur angulaire compris entre 180° et 340° et de préférence 320° ;
- la gorge a une largeur radiale sensiblement constante comprise entre 20 et 500 µm et de préférence 100 et 300 µm ;
- une profondeur axiale de la gorge mesurée entre la face distale et un point le plus profond situé dans la gorge est compris entre 20 et 500 µm et de préférence 100 et 300 µm ;
- la profondeur de la gorge est sensiblement constante ;
- la zone éclairante a un diamètre compris entre cinq et cinq cent micromètres ;
- l'orifice d'injection et l'orifice d'aspiration ont des diamètres compris entre vingt micromètres et un millimètre ;
- la zone éclairante comporte une plateforme faisant saillie de la face distale, sur une distance comprise entre 50 et 500 µm, de préférence 100 µm,
- l'embout comporte une couronne annulaire s'étendant sur le périmètre dudit embout, la couronne faisant saillie de la face distale,
- l'embout comporte une cavité avant agencée entre l'orifice d'injection et la plateforme, et, une cavité arrière agencée entre la plateforme et l'orifice d'aspiration,
- la gorge présente une profondeur axiale variable,
- la couronne, comporte un rebord annulaire prolongeant la couronne, ledit rebord annulaire étant orienté en direction de la plateforme de sorte que, le rebord annulaire surplombe partiellement l'orifice d'aspiration et/ou l'orifice d'injection.

Il est proposé en second lieu une imprimante comprenant :
- au moins un réservoir d'encre,
- au moins une pompe d'injection apte à mettre sous pression le réservoir d'encre,
- un répartiteur relié de manière fluidique au réservoir d'encre, le répartiteur étant apte à contrôler le débit d'encre prélevé du réservoir d'encre,
- un bras robotisé apte à se déplacer ou un socle robotisé apte à se déplacer,
- au moins une tête d'impression telle que précédemment décrite, montée sur le bras robotisé lorsque l'imprimante comprend un bras robotisé ou ladite au moins une tête d'impression est montée sur un support fixe de l'imprimante lorsque l'imprimante comprend un socle robotisé, l'au moins une tête d'impression étant reliée fluidiquement au répartiteur pour alimenter ladite au moins une tête d'impression en encre, ladite au moins une tête d'impression étant apte à injecter de l'encre sur un substrat,
- des moyens d'aspiration reliés fluidiquement à la tête d'impression pour aspirer de l'encre injectée par la tête d'impression,
- un réservoir d'aspiration relié fluidiquement aux moyens d'aspiration pour stocker l'encre aspirée,
- au moins une source de lumière apte à polymériser l'encre au niveau de la tête d'impression dans le cas où la tête d'impression ne comprend pas de source de lumière,
- une unité informatique connectée et contrôlant la pompe d'injection, le répartiteur, le bras robotisé ou selon le cas le socle robotisé, les moyens d'aspiration et la source de lumière.

Il est proposé en troisième lieu un procédé d'impression au moyen d'une imprimante comprenant :
- au moins un réservoir de encre,
- au moins une pompe d'injection apte à mettre sous pression le réservoir de encre,
- un répartiteur relié de manière fluidique au réservoir d'encre, le répartiteur étant apte à contrôler le débit d'encre prélevé du réservoir d'encre,
- un bras robotisé apte à se déplacer ou un socle robotisé apte à se déplacer,
- au moins une tête d'impression telle que précédemment décrite, montée sur le bras robotisé lorsque l'imprimante comprend un bras robotisé ou montée sur un support fixe de l'imprimante lorsque l'imprimante comprend un socle robotisé, l'au moins une tête d'impression étant reliée fluidiquement au répartiteur pour alimenter celle-ci en encre, la tête d'impression étant apte à injecter de l'encre sur un substrat,
- des moyens d'aspiration reliés fluidiquement à la tête d'impression pour aspirer de l'encre injectée par la tête d'impression,
- un réservoir d'aspiration relié fluidiquement aux moyens d'aspiration pour stocker l'encre aspirée,
- au moins une source de lumière apte à polymériser l'encre au niveau de la tête d'impression dans le cas où la tête d'impression ne comprend pas de source de lumière,
- une unité informatique connectée et contrôlant la pompe d'injection, le répartiteur, le bras robotisé ou selon le cas le socle robotisé, les moyens d'aspiration et la source de lumière,
dans lequel le procédé est mis en œuvre au moyen d'un programme informatique implémenté dans l'unité informatique, ce procédé comprenant :
- une étape d'injection d'au moins une encre au moyen de la tête d'impression sur un substrat immergé,
- une étape d'aspiration via la tête d'impression de l'encre,
- un étape de polymérisation au moyen de la au moins une source de lumière de l'encre injectée,
ces étapes étant réalisées simultanément pendant au moins une partie de la durée d'impression.

Diverses caractéristiques supplémentaires peuvent être prévues seules ou en combinaison :
- le procédé d'impression comprend une étape de déplacement du bras robotisé dans au moins une direction de l'espace ;
- le procédé d'impression comprend une étape de déplacement du socle robotisé dans au moins une direction de l'espace.

D'autres particularités et avantages de l'invention apparaîtront dans la description ci-après en relation avec les dessins annexés, donnés à titre d'exemples non limitatifs et dans lesquels :
- la figure 1 est une représentation schématique d'une imprimante selon l'invention,
- la figure 2 est une représentation schématique d'une extrémité d'une tête d'impression de l'imprimante de la figure 1,
- la figure 3 est une vue en perspective d'un embout de la tête d'impression,
- la figure 4a est une vue en perspective de l'embout et d'une platine de la tête d'impression,
- la figure 4b est une vue en coupe selon le plan de coupe IVb-IVb en trois dimensions de la figure 4a,
- la figure 4c est une vue en deux dimensions de l'embout selon une variante de réalisation,
- la figure 4d est une vue en perspective de l'embout de la figure 4c,
- la figure 4e est une vue en coupe selon le plan de coupe IVe-IVe de la figure 4d,
- la figure 4f est une vue en perspective d'un embout selon une autre variante de réalisation,
- la figure 4g est une vue en coupe selon le plan de coupe IVg-IVg de la figure 4f,
- le figure 5 est une image réelle illustrant le mouvement de particules,
- la figure 6 est une vue schématique en deux dimensions de l'embout,
- la figure 7 est une représentation schématique de murs imprimés par la tête d'impression selon l'invention,
- la figure 8 est une image réelle illustrant un mur imprimé en deux dimensions et en vue de dessus,
- la figure 9 est une image réelle illustrant des murs imprimés en deux dimensions et en vue de dessus,
- la figure 10 est une autre image réelle illustrant des murs imprimés en deux dimensions et en vue de dessus,
- la figure 11 est une autre image réelle illustrant des murs imprimés en trois dimensions,

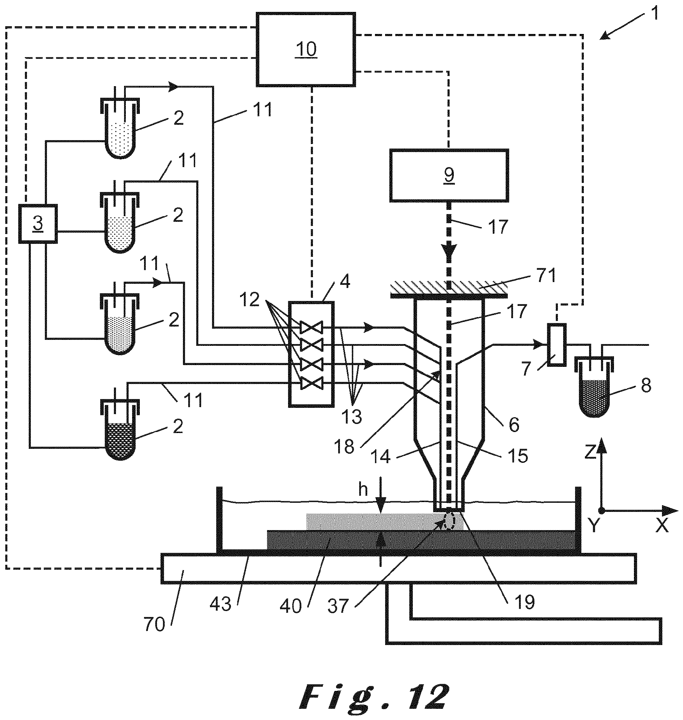

- la figure 12 est une représentation schématique de l'imprimante selon une variante de réalisation,
- la figure 13 est une vue schématique en deux dimensions d'une variante de réalisation de l'embout,
- la figure 14 est une vue schématique en deux dimensions d'une variante de réalisation de l'embout.

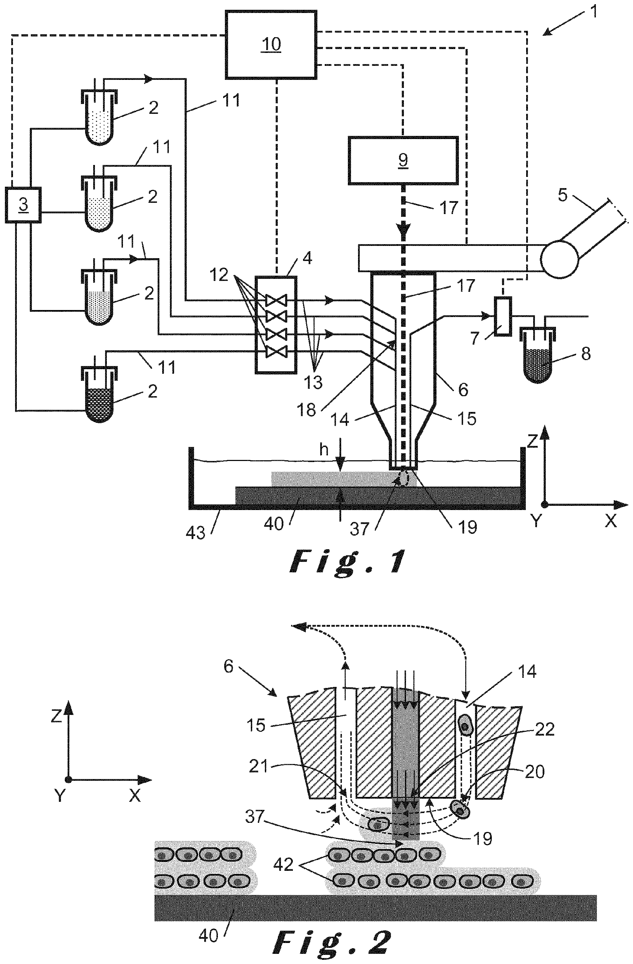

Sur la figure 1, est représentée schématiquement une imprimante 1 .

L'imprimante 1 comprend :
- au moins un réservoir 2 d'encre(s),
- au moins une pompe 3 d'injection apte à mettre sous pression le réservoir 2 d'encre,
- un répartiteur **4,**
- un bras **5** robotisé,
- une tête **6** d'impression,
- des moyens **7** d'aspiration,
- un réservoir **8** d'aspiration,
- au moins une source **9** lumière qui peut être intégrée ou non à la tête **6** d'impression, et
- une unité **10** informatique.

On définit un trièdre X, Y, Z dont l'axe X représente une première direction de déplacement horizontal du bras **5** robotisé, l'axe Y perpendiculaire à l'axe X représente une deuxième direction de déplacement horizontal et l'axe Z perpendiculaire aux axes X et Y représente une direction de déplacement vertical. Les axes X, Y et Z définissent des planx XY, XZ et YZ.

Dans le mode de réalisation représenté sur la figure 1, l'imprimante 1 comprend quatre réservoirs **2** d'encre(s). Chaque réservoir **2** comprend une encre différente.

Le nombre d'encres et par conséquent le nombre de réservoirs **2** peut être adapté en fonction des besoins, à savoir en fonction de l'hétérogénéité de la structure à imprimer.

Chaque réservoir **2** est relié de manière fluidique au répartiteur **4** au moyen d'un canal **11** d'approvisionnement.

Le répartiteur **4** comprend une pluralité de vannes **12** en nombre égal de canaux **11** d'approvisionnement. Ainsi chaque vanne **12** permet d'ouvrir ou fermer un canal **11** d'approvisionnement. Le répartiteur permet d'ouvrir et de fermer les canaux **11** d'approvisionnement en fonction des besoins d'impression. Les vannes **12** peuvent être du type tout ou rien, c'est-à-dire qu'elles sont fermées ou ouvertes de façon binaire. Ces vannes **12** peuvent aussi être à ouverture variable. Dans ce dernier cas, elles peuvent être utilisées pour ajuster le débit dans les canaux **11** d'approvisionnement à souhait.

En sortie du répartiteur, chaque canal **11** d'approvisionnement est prolongé par un canal **13** d'alimentation relié fluidiquement à la tête **6** d'impression de l'imprimante **1** . Dans un mode de réalisation non illustré sur les figures, le répartiteur **4** peut être intégré à la tête **6** d'impression.

La tête **6** d'impression est dite micro-fluidique du fait des dimensions notamment des canaux qu'elle comporte, qui sur une partie au moins de leur trajet présentent au moins une dimension transverse à l'axe, et de préférence deux dimensions transverses à l'axe inférieures au millimètre, par exemple de l'ordre de la dizaine ou de la centaine de micromètres. Les dimensions seront détaillées ultérieurement.

La tête **6** d'impression comprend :
- au moins un premier canal appelé canal **14** d'injection,
- au moins un deuxième canal appelé canal **15** d'aspiration,
- au moins une source **9** de lumière ou un guide **17** d'onde optique pour transmettre la lumière émise par une source **9** de lumière.

Les moyens **7** d'aspiration permettent de contrôler le débit ou la pression d'aspiration dans le canal **15** d'aspiration.

Dans le mode de réalisation illustré sur les figures, la tête **6** d'impression comprend un guide **17** d'onde optique.

Les canaux **13** d'alimentations sont reliés de manière fluidique au canal **14** d'injection de la tête **6** d'impression au niveau d'un embranchement **18.** Les mélanges d'encres sont ainsi réalisés dans la tête **6** d'impression. Toutefois, les mélanges d'encresx peuvent être réalisés à un autre niveau. Par exemple, il est possible que l'embranchement **18** se situe à l'extérieur de la tête **6** d'impression.

En référence à la figure 2, le canal **14** d'injection et le canal **15** d'aspiration débouchent sur une face **19** distale de la tête d'impression. La face **19** distale est située à une extrémité de la tête **6** d'impression. Le canal **14** d'injection et le canal **15** d'aspiration débouchent respectivement sur la face **19** distale par un premier orifice appelé orifice **20** d'injection et un deuxième orifice appelé orifice **21** d'aspiration. La source **9** de lumière est apte à éclairer une zone 22 éclairante située sur la face **19** distale. La zone 22 éclairante est une zone délimitée illuminée de préférence par une source **9** de lumière apte à générer un faisceau de lumière collimaté par exemple. Une source **9** de lumière apte à générer un faisceau de lumière, de préférence cohérent. D'autres faisceaux peuvent être aussi utilisées. A titre d'exemples, des sources lumineuses telles que des LED, diodes lasers, barres CCD ou VCSEL peuvent être utilisées. La zone **22** éclairante est avantageusement protégée par un matériau transparent à l'inverse de l'orifice **20** d'injection et de l'orifice **21** d'aspiration, ceci pour protéger la source **9** de lumière afin que de l'encre ne la détériore pas.

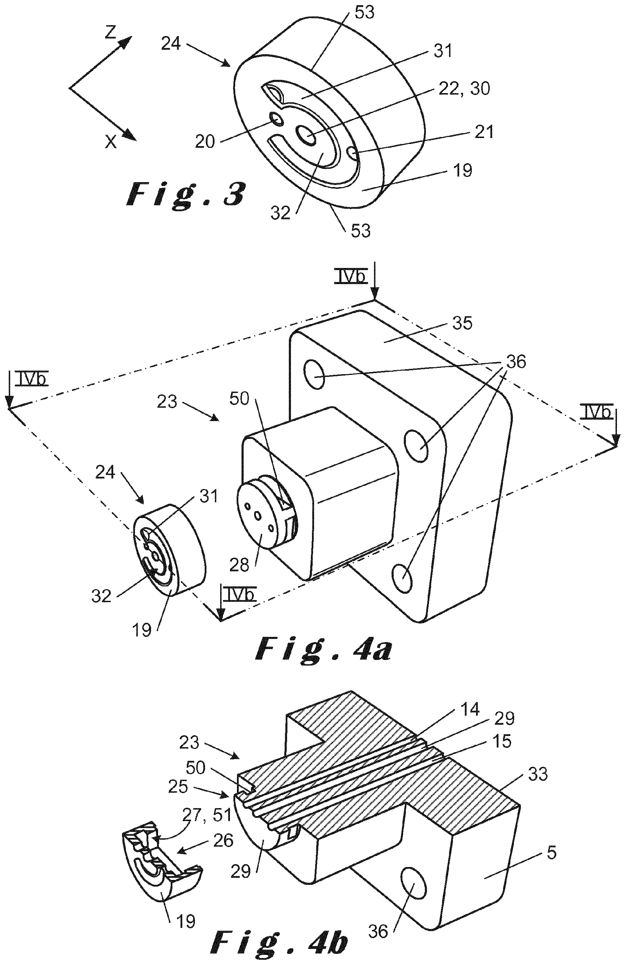

En référence aux figures 4a et 4b, la tête **6** d'impression comprend un corps **23** et un embout **24.** Ceci permet avantageusement de produire un tête **6** d'impression avec deux matériaux différents, notamment en ce qui concerne l'embout **24** qui a des spécificités particulières ainsi qu'expliqué ultérieurement.

Selon un mode de réalisation préféré, l'embout **24** présente une forme cylindrique dont le diamètre est compris entre cent micromètres et cinq millimètres. D'autres formes d'embout **24** sont possibles en variantes.

Selon un mode de réalisation préféré, l'embout **24** peut-être rapporté sur le corps puis fixé à ce dernier. Divers moyens de fixation peuvent être envisagés. Dans le mode de réalisation illustré, le corps **23** comprend une portion **25** d'extrémité munie des encoches **50** tandis que l'embout **24** définit une cavité **26** creuse dont des parois **27** internes comportent des pions **51** aptes à s'insérer dans les encoches **50.** L'embout **24** est rapporté et fixé sur la portion **25** d'extrémité grâce aux pions **51** logeant dans les encoches **50.** La portion **25** d'extrémité est munie d'une face **28** intermédiaire sur laquelle débouchent le canal **14** d'injection, le canal **15** d'aspiration et un troisième canal appelé canal **29** optique. L'embout **24** comprend la face **19** distale, l'orifice **20** d'injection, l'orifice **21** d'aspiration et la zone **22** éclairante. Sur les figures 3, 4a et 4b, la zone 22 éclairante se présente sous la forme d'un troisième orifice appelé orifice **30** optique traversant de part et d'autre ledit embout **24** et débouchant sur la face **19** distale à l'instar de l'orifice **20** d'injection et de l'orifice **21** d'aspiration. Lorsque l'embout **24** est monté sur la portion **25** d'extrémité du corps **23,** l'orifice **20** d'injection, l'orifice **21** d'aspiration et l'orifice **30** optique sont positionnés respectivement en regard du canal **14** d'injection, du canal **15** d'aspiration et du canal **29** optique du corps **23.**

Dans une variante de réalisation, l'embout **24** est transparent. Dans ce cas, l'embout **24** ne comporte pas d'orifice **30** optique.

La tête **4** d'impression est monobloc. La tête **4** d'impression peut comprendre plus de deux matériaux différents. Dans le cas d'une tête **4** d'impression monobloc, celle-ci est avantageusement transparente pour permettre le passage de la lumière.

Avantageusement la face **19** distale est munie d'une gorge **31** axiale. La gorge **31** comprend au moins en partie l'orifice **21** d'aspiration. Dans le mode de réalisation représenté sur les figures, l'orifice **21** d'aspiration est inscrit dans la gorge **31.** En d'autres termes l'orifice **21** d'aspiration est intégralement contenu dans la gorge **31.** Dans un mode préféré, la gorge **31** présente une forme en arc de cercle autour d'un centre et s'étend sur la circonférence de l'embout **24** selon un secteur angulaire compris entre 180° et 340°, de préférence sensiblement égal à 320°. Ainsi la gorge **31** n'est pas fermée. Des expérimentations ont permis de mettre en évidence qu'un angle de 320° permet avantageusement de canaliser à l'aspiration l'intégralité de l'encre injectée. Selon des variantes de réalisation non illustrées sur les figures, la gorge **31** n'est pas limitée à une forme en arc de cerle. Celle-ci peut par exemple être en forme d'arc d'ellipse. Elle peut également avoir une autre forme permettant de préférence d'entourer la zone 22 éclairante.

La gorge **31** présente une largeur radiale (selon un axe radial X de l'embout) sensiblement constante. La largeur radiale est de préféfence comprise entre 20 et 500 µm et de préférence entre 150 et 250 µm.

La gorge **31** a une profondeur axiale (selon l'axe Z) mesurée entre la face **19** distale et un point le plus profond situé dans la gorge **31** de préférence comprise entre 20 µm et 500 µm, de préférence entre 150 et 250 µm. De préférence, également la gorge **31** présente une profondeur sensiblement constante et/ou une section sensiblement constante.

Une telle profondeur et une telle largeur radiale de la gorge **31** permettent à cette dernière d'assurer un récupération optimale de l'encre injectée.

La gorge **31** définit une zone **32** centrale. L'orifice **20** d'injection est avantageusement situé dans la zone **32** centrale. Ainsi lorsque de l'encre est expulsé de la tête **6** d'impression par l'orifice **20** d'injection, la part de cette encre qui n'est pas polymérisée est avantageusement canalisée par la gorge **31,** et ensuite aspirée par l'orifice **21** d'aspiration. Le mouvement de l'encre sera décrit ultérieurement.

La gorge **31** peut en section (section dans un plan de coupe XZ) prendre la forme d'un carré, d'une ellipse, d'une pyramide ou toute autre forme mais est de préférence cylindrique.

La zone **22** éclairante se situe avantageusement entre l'orifice **20** d'injection et l'orifice **21** d'aspiration. De manière plus précise, les orifices **20, 21** et l'orifice **30** optique sont alignés. En d'autres termes, chaque orifice **20, 21, 30** comprennent un centre (non représenté sur les figures) de sorte qu'une droite (non représentée) passe sensiblement par chacun de ces centres. Toutefois, il est possible que ces centres ne soient pas alignés, c'est par exemple le cas lorsque la tête **6** d'impression comprend plusieurs orifices **20, 21, 30** d'injection, d'aspiration et optique. Cette variante, non représentée sur les figures, sera décrite ultérieurement.

Le canal **15** d'aspiration est relié fluidiquement aux moyens **7** d'aspiration et au réservoir **8** d'aspiration. Un moyen **7** d'aspiration peut par exemple être une pompe **7** d'aspiration.

Dans le mode de réalisation représenté sur les figures 1 à 4, la source **9** de lumière utilisée est une source **9** de lumière cohérente, par exemple un laser **9.** La puissance du laser **9** est comprise entre 0,1 mW et 160 mW, et de préférence entre 1 mW et 10mW. La puissance du laser **9** émise correspondant à la quantité d'énergie par unité de surface est de 0.8 mW pour une aire circulaire exposée de 20µm de diamètre. L'éclairement est compris entre 0,3.10⁶ W.m⁻² et 500.10⁶ W.m⁻² et de préférence entre 1.10⁶ et 75.10⁶ w.m⁻². La longueur d'onde est comprise entre 265 nm et l'infrarouge et de préférence entre 365 nm et 750 nm. Le laser 9 est situé en dehors de la tête **6** d'impression ainsi qu'illustré schématiquement sur la figure 1. La source **9** de lumière peut être une diode laser, une LED, une VCSEL. D'autres outils peuvent être utilisés. De préférence la zone 22 éclairante (et par conséquent le faisceau lumineux) a un diamètre compris entre cinq et cinq cent micromètres.

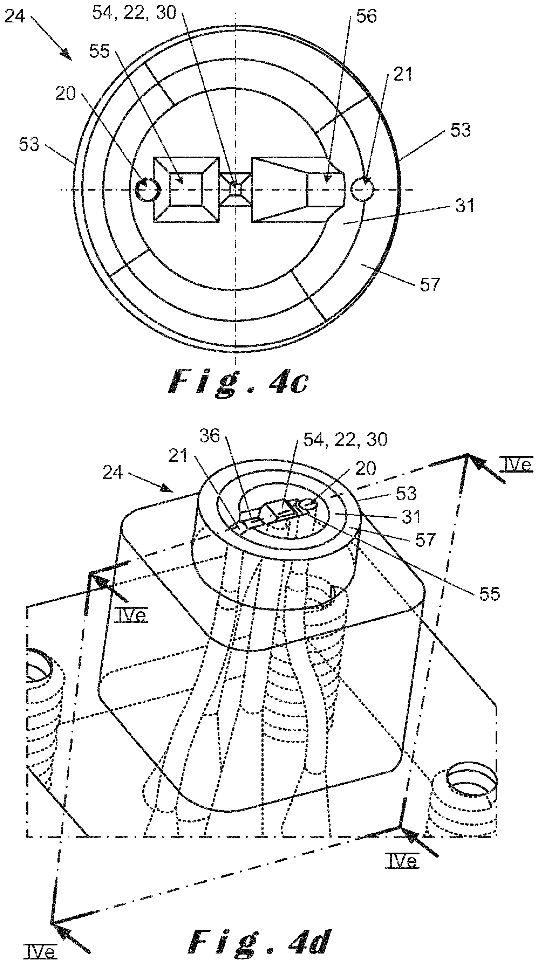

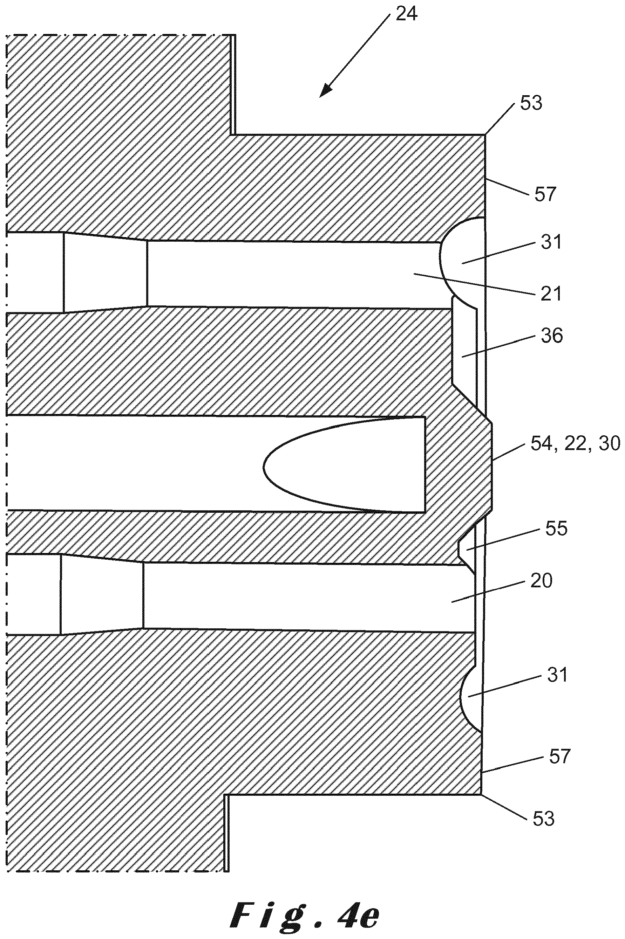

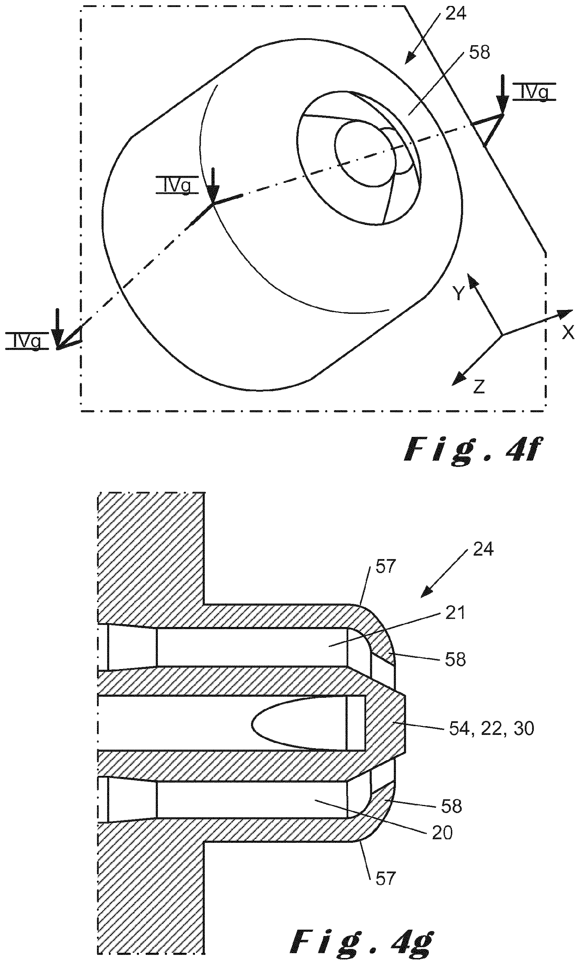

Dans ce mode de réalisation le canal **29** optique pratiqué dans la tête **6** d'impression s'étend depuis une surface **33** supérieure de celle-ci jusqu'à la face **28** intermédiaire. La tête **6** d'impression comprend le guide **17** d'onde optique inséré dans le canal **29** optique et permettant de guider le faisceau cohérent du laser **9** jusqu'à l'orifice **30** optique. En référence à la figure 4, une extrémité inférieure (non représentée sur les figures) du guide **17** d'onde optique est située au niveau de la face **28** intermédiaire. Avantageusement le guide **17** d'onde optique ne s'étend pas jusqu'à l'orifice **30** optique. L'extrémité inférieure du guide **17** d'onde optique comprend une lentille, par exemple une lentille sphérique, qui permet à la fois de protéger le guide **17** d'onde optique et d'obtenir un meilleur faisceau de lumière. Le guide **17** d'onde optique est par exemple une fibre optique. Dans une variante de réalisation, le guide **17** d'onde optique peut ne pas être muni d'une lentille.

Dans une variante de réalisation non illustrée sur les figures, l'orifice **30** optique peut être muni d'une lentille supplémentaire en fonction du besoin.

Ainsi que précédement évoqué, l'imprimante **1** comprend une unité **10** informatique permettant de coordonner les différents éléments que celle-ci comprend. L'unité **10** informatique peut être un ordinateur, microprocesseur, ou plus généralement tout moyen de contrôle automatisé des opérations électroniques, optiques, mécaniques et/ou fluidiques. Ainsi l'unité **10** informatique peut être connectée de façon filaire ou sans fils à la pompe **3** d'injection, au répartiteur **4,** au bras **5** robotisé, à la source **9** de lumière, à la pompe **7** d'aspiration. Ces connexions sont représentées schématiquement en traits pointillés sur la figure 1. L'unité informatique comprend un processeur dans lequel est implémenté un programme d'ordinateur pour la mise en œuvre d'un procédé d'impression.

L'expression pompe n'est nullement limitative, il peut s'agir de tout moyen capable de comprimer ou de transporter un fluide, ou de créer une dépression (aspirer).

Le corps de la tête **6** d'impression comprend une platine **35** munie d'une pluralité de trous **36** de fixation. Ces trous **36** sont destinés à accueillir des vis de fixation (non représentées). La platine **35** est avantageusement fixée sur le bras **5** robotisé. Le bras **5** robotisé peut se déplacer dans les trois directions de l'espace. Il permet ainsi à la tête **6** d'impression de fabriquer des structures tridimensionnelles.

Le corps est avantageusement fabriqué en polymère. Le corps **23** est par exemple fabriqué par usinage. D'autres matériaux peuvent être utilisés.

Selon un mode de réalisation préféré, l'embout **24** est réalisé en polymère, de préférence un polymère transparent. De préférence également, ce polymère transparent est un élastomère par exemple du Poly(DimethylSiloxane). Il est fabriqué par moulage à l'aide d'un moule micro-usiné (non représenté). La tête d'impression peut également être fabriquée dans ce matériau. La tête d'impression et l'embout 24 peuvent ainsi être fabriqués dans un matériau identique ou dans des matériaux différents.

Le matériau avec lequel est fabriqué l'embout **24** présente une importance particulière dans la mesure où c'est ce matériau qui est en contact avec une zone **37** de polymérisation. La zone **37** de polymérisation est la zone où la structure tridimensionnelle est fabriquée.

Avantageusement, le(s) matériau(x) qui compose(nt) la tête est(sont) :
- inerte(s) vis-à-vis de la réaction de polymérisation,
- de préférence mais pas nécessairement transparent(s) afin de permettre à des rayons lumineux émis par la source lumineuse de traverser le matériau au niveau de la zone éclairante,
- de préférence poreux aux gaz, notamment à l'oxygène afin d'inhiber localement la réaction de polymérisation sur une faible épaisseur de l'ordre du micromètre et d'éviter que le matériau polymérisé ne colle à l'embout **24** pendant l'impression.

L'embout **24** peut être réalisé dans un polymère déformable élastomère, un polymère rigide ou encore un métal, par exemple le polydiméthilsiloxane (PDMS) qui présente l'avantage d'être inerte, transparent, déformable et du fait de sa porosité à l'oxygène empêche la polymérisation et l'adhésion de l'encre sur la surface. Dans le cas où l'embout **24** n'est pas réalisé dans un matériau transparent (comme c'est le cas dans le mode de réalisation représenté sur les figures), il convient de créer un orifice **30** optique au niveau de la zone 22 éclairante (ainsi que précédemment évoqué) et d'y insérer une fenêtre transparente afin de protéger la source de lumière. Dans ce cas, la fenêtre transparente peut être une lamelle plane ou bien une lentille, par exemple une lentille sphérique pour obtenir un faisceau lumineux de préférence collimaté ainsi que précédemment expliqué.

A noter que la tête peut être fabriquée entièrement avec un même matériau.

Comme précédemment évoqué, la source **9** de lumière peut être intégrée à la tête **6** d'impression ou être distincte. Dans le dernier cas la tête **6** d'impression comprend un guide **17** d'onde optique telle qu'une fibre optique pour acheminer les rayons lumineux au travers de la tête **6** d'impression jusqu'à la zone **22** éclairante. Afin de s'affranchir du guide **17** d'onde optique, une variante de réalisation peut consister à positionner la source **9** de lumière près de la face **19** distale.

L'orifice **20** d'injection et l'orifice **21** d'aspiration présentent un diamètre qui varie entre vingt micromètres et un millimètre. Les diamètres du canal **14** d'injection et du canal **15** d'aspiration sont également compris entre vingt micromètres et un millimètre. Ces dimensions sont avantageuses puisqu'elles permettent de protéger des cellules **42** contre des forces de cisaillement. L'orifice **20** d'injection et l'orifice **21** d'aspiration peuvent présenter en variantes une section de forme carrée ou rectangulaire, ou toute autre forme. Une forme préférée est cependant circulaire.

La pression d'injection dans le canal **14** d'injection est de préférence comprise entre zéro et un bar. Cette pression est obtenue en pressurisant les réservoirs **2** à l'aide de la pompe **3** d'injection. La pression d'aspiration est comprise entre moins un bar (dépression) et zéro bar. Cette pression d'aspiration est obtenue au moyen de la pompe **7** d'aspiration. Avantageusement le débit d'aspiration (dépression) en valeur absolue est supérieur au débit d'injection en valeur absolue pour éviter la contamination du milieu environnant autour de la tête entre l'orifice **20** d'injection et l'orifice **21** d'aspiration et favoriser la collecte des encres injectées par l'orifice **20** d'injection.

La tête **6** d'impression présente une vitesse de déplacement selon l'axe X et selon l'axe Y qui peut aller jusqu'à un centimètre par seconde. L'impression est en générale réalisée selon l'axe X et selon l'axe Y avec des mouvements simultanés ou isolés selon ces axes. La tête peut également se déplacer selon l'axe X,l'axe Y et l'axe Z de manière simultanée ou effectuer un mouvement dans le plan XY puis un mouvement selon l'axe Z et inversement. La vitesse de la tête **6** d'impression selon l'axe Z peut aller également jusqu'à dix centimètres par seconde.

Une distance séparant la tête **6** d'impression d'un substrat **40** est comprise entre 10 µm et 800 µm. A noter qu'en dessous de 20 µm la circulation du fluide est difficile et au-delà de 400 µm, l'efficacité de la collecte des encres par aspiration diminue induisant un risque de contamination du milieu environnant. De façon préférée, cette distance est comprise entre 40 µm et 200 µm.

Dans ce qui suit, le fonctionnement de l'imprimante va être décrit. Ainsi que précédemment expliqué la tête **6** d'impression se déplace à l'aide d'un bras **5** robotisé contrôlé par l'unité **10** informatique. Une structure tridimensionnelle est créée à l'aide d'un outil de conception assisté par ordinateur et importée dans l'unité **10** informatique. La structure peut par exemple être contenue dans un fichier informatique. La structure peut être hétérogène (plusieurs encres) ou simple (une seule encre). L'hétérogénéité de la structure peut notamment être obtenue en injectant localement différentes encres, en modifiant la longueur d'onde et/ou de l'intensité de la source de lumière au cours de l'impression.

Le substrat **40** qui supportera la structure est plongé dans un liquide, avantageusement une solution aqueuse qui constitue la zone de polymérisation, le tout est contenu dans une boite de Pétri **43** par exemple. Dans d'autres modes de réalisation, la zone de polymérisation peut contenir un autre liquide, par exemple, une huile. Dans un mode préféré, la tête **6** d'impression est positionnée de sorte à ce que la face **19** distale de l'embout **24** baigne dans le mélange d'eau et d'hydrogel.

L'impression débute lorsque l'unité **10** informatique donne l'ordre au répartiteur **4** d'ouvrir au moins une vanne **12** d'ouverture. Les encres correspondantes sont alors acheminées vers la tête **6** d'impression. Les encres passent par le canal **14** d'injection et est (sont) ejecté(es) de la tête **6** d'impression par l'orifice **20** d'injection. Les encres sont ejectées dans toutes les directions qui s'offrent à elles. En parallèle la pompe **7** d'aspiration est activée. La dépression dans le canal **15** d'aspiration est supérieure en valeur absolue à la pression d'injection dans le canal **14** d'injection. Les encres éjectées par le canal **14** d'injection se dirigent vers le canal **15** d'aspiration. La part des encres qui se dirige vers le canal **15** d'aspiration par le chemin le plus court qui s'offrent à elles en passant sous la zone **22** éclairante est polymérisée. L'activation de la ou des sources lumineuses engendre la polymérisation localisée des encres, la tête est alors mise en mouvement. La source lumineuse est activée au cours du déplacement lorsque la position de la tête relative à l'échantillon atteint la zone à polymériser. Cete encre polymérisée se fixe sur le substrat **40** préalablement disposé en regard de la face **19** distale de la tête **6** d'impression. En même temps une autre part de l'encre éjectée est perdue. Cette encre perdue est canalisée par la gorge **31** puis aspirée par le canal **15** d'aspiration via l'orifice **21** d'aspiration. Cette encre n'est pas polymérisée car elle contourne la zone **22** éclairante.

A noter que le débit d'aspiration est plus important que le débit d'injection, par conséquent, l'ensemble de l'encre injectée est aspirée en même temps que de la solution environnante.

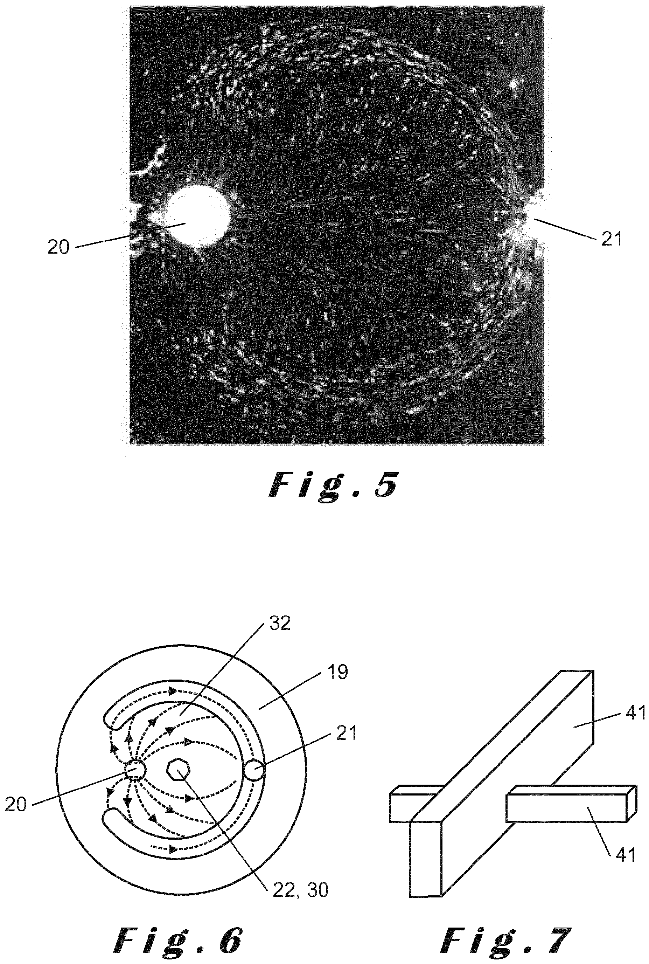

En référence à la figure 5, des micro-particules fluorescentes de dix micromètres ont été utilisées pour illustrer les écoulements de l'encre. Dans cette expérience, la pression d'injection est de soixante millibar et la pression d'aspiration est de quatre-vingt-dix millibar. Le mélange d'eau et d'hydrogel est respectivement réalisé dans des proportions de vingt-cinq et soixante-quinze pourcent.

La structure de l'embout **24,** notamment grâce à la gorge dans laquelle est inscrit l'orifice **21** d'aspiration, permet avantageusement de canaliser l'encre ainsi que ceci est visible sur les figures 5 et 6. Sur la figure 6 notamment le mouvement d'encre est schématisé par des lignes de flux flechées. Comme on peut le voir, la quasi-totalité de l'encre est canalisée par la gorge et aspirée. Ce mécanisme permet d'éviter la contamination du milieu environnant et de favoriser le renouvellement ou le changement d'encre au niveau de la zone de polymérisation.

Sur la figure 4c est représentée une variante de réalisation de la tête d'impression. Dans ce mode de réalisation, la profondeur axiale de la gorge **31** varie. Plus précisément, la profondeur axiale de la gorge **31** varie selon que l'on se rapproche de l'orifice **21** d'aspiration. Ceci augmente la résistance microfluidique dans la gorge **31** ce qui permet à plus d'encre de passer sous l'orifice **30** optique. L'impression est donc plus efficace.

Dans ce mode de réalisation, la zone **22** éclairante comporte une plateforme **54.** La plateforme **54** fait saillie de la face **19** distale de l'embout **24,** selon l'axe Z. La hauteur de la plateforme **54,** selon l'axe Z, est comprise entre 50 et 500 µm et de préférence, de 100 µm. Une hauteur de 100 µm permet d'obtenir un bon compris entre l'aspiration du matériau et la vitesse de déplacement de la tête d'impression. La plateforme **54** permet d'éloigner l'embout de la zone **37** de polymérisation afin que des bords **53** périphériques de l'embout **24** ne heurtent pas l'encre polymérisée. La plateforme **54** présente avantageusement une forme de pyramide tronquée. D'autres formes peuvent être utilisées tel qu'un cube par exemple. Toutefois, un mode préfére de réalisation comporte une plateforme en forme de pyramide tronquée. En effet, des études menées sur les écoulement autour de la plateforme **54** par la demanderesse, indiquent que l'utilisation d'une pyramide tronquée permet avantageusement d'éviter la présence de zones de faibles écoulement qui peuvent être à l'origine de contaminations lors des changements d'encres.

Dans ce mode de réalisation, l'embout **24** comporte, une cavité **55** avant agencée entre l'orifice **20** d'injection et la plateforme **54.** L'embout **24** comporte une cavité **56** arrière agencée entre la plateforme **54** et l'orifice **21** d'aspiration. Les cavités **55, 56** avant et arrière présentent une forme sensiblement pyramidale. Elles sont creusées à l'intérieur de l'embout selon l'axe Z. Elles permettent avantageusement de créer un espace fluidique entre l'orifice **20** d'injection et la plateforme **54** d'une part, et entre la plateforme **54** et l'orifice **21** d'aspiration d'autre part. Ceci diminue la résistance microfluidique, ce qui favorise l'ecoulement sous la plateforme **54** permettant d'améliorer le renouvellement de l'encre. Des cavités en forme de pyramides tronquées, permettent avantageusement d'éviter des variations brutes de hauteur dans lesdites cavités. D'autres formes peuvent être utilisées tel que des cavités hemicylindriques par exemple. En effet, des variations brutes de hauteur dans la cavité auraient un impact négatif sur le flux d'encre.

Dans ce mode de réalisation, l'embout comporte une couronne **57.** La couronne **57** est annulaire autour de l'axe Z. Elle s'étend sur le périmètre de l'embout **24.** La couronne **57** fait saillie de la face **19** distale. La couronne **57** ne dépasse pas la plateforme **54** selon l'axe Z. En d'autres termes, la plateforme s'étend, au plus, au niveau de la plateforme **54,** selon l'axe Z.

L'embout **24** peut être muni de la couronne **57** et/ou la plateforme **54** et/ou cavité **55** avant et/ou cavité **56** arrière et/ou de la gorge **31** à profondeur variable.

Dans une variante de réalisation représentée sur les figures 4f et 4g, la couronne **57** comporte un rebord **58** annulaire. Le rebord **58** annulaire prolonge la couronne **57** en direction de la plateforme **54,** c'est-à-dire selon l'axe X et l'axe Y. Ainsi le rebord **58** annulaire surplombe partiellement l'orifice **21** d'aspiration et l'orifice **20** d'injection.. En effet, la tête est positionné à une certaine distance du substrat afin d'avoir une zone de confinement qui permet une bonne aspiration de l'encre par l'orifice d'aspiration. En éloignant la tête du substrat, la zone de confinement est compromise, et donc l'aspiration de l'encre l'est aussi ce qui nuit à l'impression. Le rebord **58** annulaire permet de reproduire une zone de confinement et de maintenir une bonne aspiration, il en résultat qu'il est possible d'éloigner la tête d'impression du substrat pour imprimer des structures plus hautes, selon l'axe Z, et/ou d'augmenter la vitesse d'impression (déplacement selon les axes X et/ou Y).

Ainsi que précédemment évoqué, l'encre est polymérisée en passant devant la zone **22** éclairante suivant des dimensions latérales (X,Y) définies par la forme du faisceau. L'épaisseur de la structure polymérisée est définie par confinement géométrique par la distance entre la face distale et le substrat **40** ou une structure préalablement imprimée. L'encre est alors réticulée sur le substrat **40** ou sur une structure préalablement imprimée. La face **19** distale de la tête **6** d'impression est positionnée à une certaine distance **h** du substrat **40** ou d'une structure préalablement imprimée. Cette distance correspond à la hauteur maximale d'une couche que la tête **6** d'impression peut imprimer lors de l'illumination. La résolution en X,Y des structures imprimées est définie principalement par les propriétés optiques du faisceau lumineux induisant la polymérisation qui garantissent une précision donnée pour des épaisseurs de couches comprises entre 0 et h. Par exemple, lorsque l'on souhaite constuire une structure ayant une hauteur de six cent micromètres sur une longeur donnée et que la capacité d'impression de la tête **6** d'impression est de deux cent micromètres de haut avec une précision acceptable, la face **19** distale sera premièrement située à une distance de deux cent micromètres.L'encre est polymérisée en créant un premier étage de la structure de deux cent micromètres de haut et en déplaçant latéralement le bras **5** robotisé dans le plan (X,Y) souhaité pour fabriquer la structure souhaitée. Une fois la longeur donnée atteinte, le bras **5** robotisé est déplacé selon l'axe Z d'une distance de deux cent micromètres et suit le chemin emprunté en sens inverse pour construire un deuxième étage et ainsi de suite jusqu'à atteindre une hauteur de six cent micromètres, dans le cas présent trois étages successifs.

Un exemple schématisé de murs **41** se croisant est représenté sur la figure 7. Sur cette figure, les murs **41** qui se croisent présentent des hauteurs différentes. La figure 7 illustre schématiquement que ce procédé et cette imprimante **1** permettent d'empiler des structures réalisées en encres au niveau du croisement. Des expériences réalisées par la demanderesse ont permis de démontrer que les empilements de structures sont mieux réalisés en utilisant une source de lumière collimatée qui permet d'augmenter la valeur h, c'est-à-dire qui permet de maintenir la précision de polymérisation sur de plus grandes épaisseurs de polymérisation. L'utilisation d'un faisceau de lumière focalisé est possible, toutefois un faisceau de lumière collimaté est préféré.

L'imprimante qui vient d'être décrite permet de réaliser des structures complexes avec des discontinuités, des hauteurs différentes, des croisements de murs et des empilements. Elle permet de réaliser des structures complexes comprenant des cavités creuses. Elle offre ainsi une grande liberté de création aux scientifiques pour reproduire un environnement de croissance des cellules.

Un procédé de mise en œuvre de l'imprimante comprend :
- une étape d'injection d'une ou plusieurs encre(s) sur un substrat immergé, l'injection étant réalisée via le canal **14** d'injection,
- une étape d'aspiration de ou des l'encre(s) via le canal **15** d'aspiration,
- une étape de polymérisation de ou des l'encre(s) injectée(s).

Ces étapes peuvent être en partie simultannées, et sont de préférence simultannées pendant au moins une partie du temps d'impression. Elles peuvent également de préférence être réalisées en même temps que la tête **6** d'impression se déplace dans l'espace suivant une direction ou simultanément selon plusieurs directions (X,Y,Z). Au cours de l'impression, une extrémité de la tête d'impression est immergée dans la zone **37** de polymérisation

L'imprimante 1 ci-dessus décrite présente de nombreux avantages parmi lesquels :
- elle offre la possibilité de créer des structures hétérogènes tout en ayant une résolution au moins inférieur à 20 micromètres grâce à la précision du faisceau lumineux dont le diamètre peut être suffisamment bas pour obtenir des hautes résolutions,
- elle permet de contrôler de façon précise la composition du mélange des encres utilisées,
- les flux des encres peuvent être contrôlés dans le temps et dans l'espace,
- les quantités des encres utilisées sont minimisées,
- elle peut imprimer dans les trois directions de l'espace et ceci avec une résolution de l'ordre du micromètre,
- elle peut imprimer avec différentes longueurs d'ondes de manière séquentielle et/ou simultanément,
- elle permet d'imprimer selon différentes résolutions.

Dans une variante de réalisation (non illustrée) de la tête d'impression, cette dernière peut comprendre une pluralité de canaux d'injection, de canaux d'aspiration et de zones éclairantes.

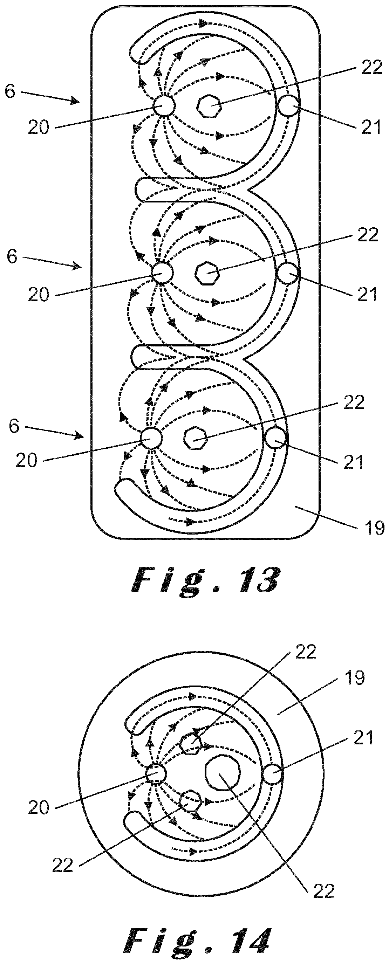

Dans une autre variante de réalisation illustrée sur la figure 13, l'imprimante peut être équipée de plusieurs têtes **6** d'impression et de plusieurs zones **22** éclairantes. La longueur d'onde et le diamètre des sources lumineuses peut varier d'une tête d'impression à une autre.

Dans une autre variante de réalisation illustrée sur la figure 14, une tête **6** d'impression comprend plusieurs zones **22** éclairantes. Dans ce mode de réalisation, la tête d'impression comprend trois zone éclairantes dont la longueur d'onde et le diamètre peut varier l'une par rapport à l'autre.

Dans une autre variante de réalisation illustrée sur la figure 14, la tête **6** d'impression comprend une gorge 31 annulaire qui s'étend sur toute la périphérie de la tête d'impression ainsi qu'une pluralité de zones 22 éclairantes. Les zones éclairantes peuvent avoir une longueur d'onde et un diamètre qui varient d'une zone éclairante à l'autre.

Dans une autre variante de réalisation non illustrée sur les figures, la tête d'impression comporte un canal d'injection et deux canaux d'aspiration. Avantageusement, la gorge est annulaire. Des essais réalisés par la demanderesse démontre qu'une telle tête d'impression est avantageuse en ce que la récupération de l'encre au travers des canaux d'aspiration est efficace.

Dans une autre variante de réalisation (non illustrée), le tête d'impression est fixe et la boîte de Pétri 43 est montée sur un socle robotisé contrôlé par l'unité 10 informatique. Dans ce mode de réalisation, la tête d'impression est montée sur un support fixe et seul le socle est en mouvement pour former les structures à imprimer. Dans cette variante de réalisation, la tête 6 d'impression ne se déplace pas et les étapes précédemment décrites du procédé sont réalisées en même temps que le socle robotisé se déplace.

Dans ce qui suit, vont être décris plusieurs tests réalisés en laboratoire.

Ces expérimentations ont été obtenues avec une impression dans un hydrogel synthétique PEG DA (Poly Ethylène Glycol Diacrylate). L'impression a été réalisée sur une lamelle en verre fonctionalisée avec du MAPTMS (Amino Propyl Tri Methoxy Silane), plongée dans l'hydrogel synthétique.

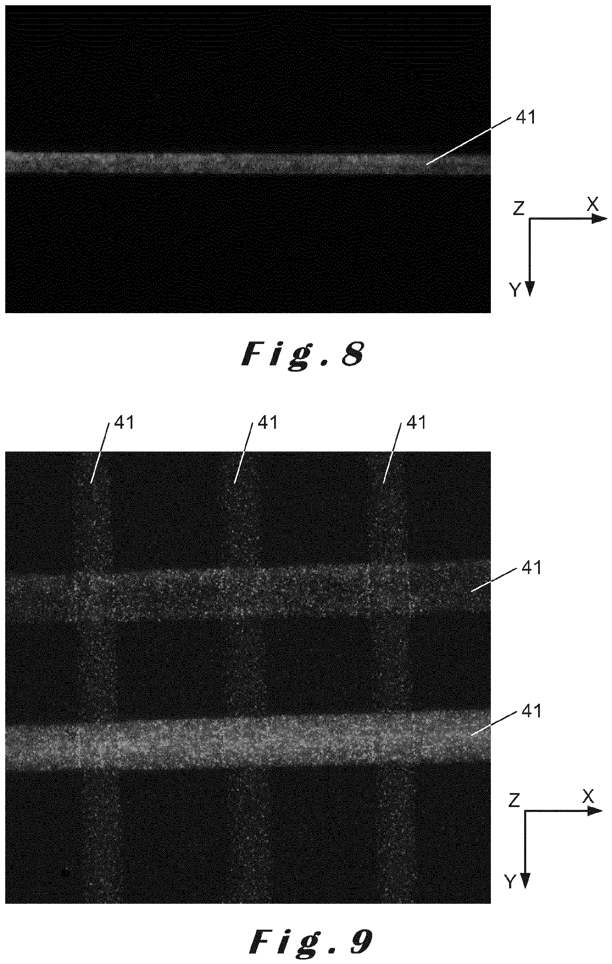

### Test 1 (figure 8) : Structure en hydrogel imprimée sur un substrat de verre.

Encre: 50% PEG DA (Mw 700) 50% d'eau 0,0075% d'Irgacure 819 2% particules fluorescentes (diamètre 300 nm)
Distance séparant la face distale du substrat : 60µm
Pression d'injection : 100 mbar
Pression d'aspiration : -150 mbar
Vitesse selon l'axe X : 0,1 mm/s
Puissance du laser : 4 mW
Résolution : 20µm (largeur selon l'axe Y)
Longueur d'onde : 405 nm

Dans le test 1, une ligne horizontale d'une hauteur de 60 µm est tracée.

### Test 2 (figure 9) : Structure en hydrogel imprimée sur un substrat de verre.

Encre: 50% PEG DA (Mw 700) 50% d'eau 0,0075% d'Irgacure 819 2% particules fluorescentes (diamètre 300 nm)
Distance séparant la face distale du substrat : 60µm, 120µm, 140µm
Pression d'injection : 100 mbar
Pression d'aspiration -150 mbar
Vitesse selon l'axe X : 0,1 mm/s
Puissance du laser : 4 mW
Résolution : 50µm (largeur selon l'axe Y)
Longueur d'onde : 405 nm

Dans le test 2, trois lignes verticales sont tracées avec une distance séparant la face distale du substrat de 60 µm.

Puis la face distale est positionnée à une distance de 120 µm. Une première ligne horizontale est tracée.

La face distale est ensuite positionnée à une distance de 140 µm. Une seconde ligne horizontale est tracée.

Cet exemple permet de montrer qu'il est possible d'empiler des couches d'épaisseurs variables les unes sur les autres. En effet, les résultat obtenus sont particulièrement prometteurs et démontrent que les structures obtenues sont stables.

En effet, à l'intersection entre la première ligne horizontale et l'une quelconque des lignes verticales, l'empilement d'un premier étage et d'un deuxième étage est réalisée et la structure est stable.

Le même constat est fait en ce qui concerne la deuxième ligne horizontale.

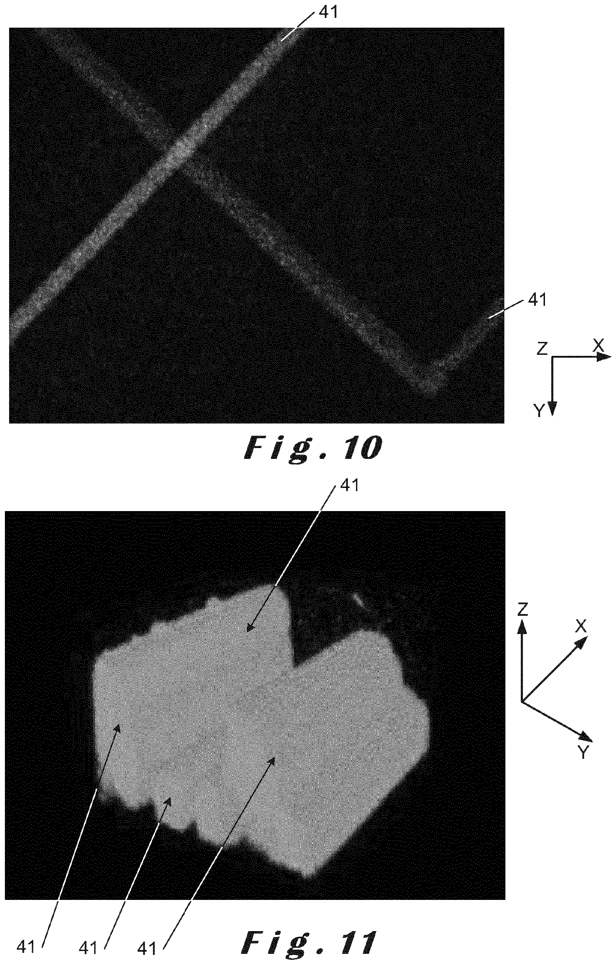

### Test 3 (figure 10) : Structure en hydrogel imprimée sur un substrat de verre.

Encre: 50% PEG DA (Mw 700) 50% d'eau 0,0075% d'Irgacure 819 2% particules fluorescentes (diamètre 300 nm)
Distance séparant la face distale du substrat : 60µm
Pression d'injection : 100 mbar
Pression d'aspiration -150 mbar
Vitesse selon l'axe X : 0,1 mm/s
Puissance du laser : 2 mW
Résolution : 20µm (largeur dans le plan XY)

Dans le test 3, la distance séparant la face distale du substrat est initialement de 60 µm. Une première ligne oblique est tracée, sa longeur est de 1 mm et sa largeur de 30µm.

La distance séparant la face distale du substrat est ensuite augmentée de 40µm ce qui la porte à 100 µm. Une deuxième ligne oblique d'une hauteur de 100 µm est tracée, d'une longueur de 1 mm et d'une largeur de 40µm.

La largeur augmente du fait de l'éloignement de la zone éclairante du substrat. Les rayons lumineux émis par la zone éclairante ne peuvent être parfaitement collimatés et présentent par conséquent une légère divergence.

Le test 3 permet d'illustrer qu'il est possible d'une part de tracer des lignes obliques et que ces structures sont stables lorsqu'elles sont empilées les unes sur les autres ainsi que ceci est visible au niveau du croisement de ces deux lignes.

### Test 4 (figure 11) : Structure en hydrogel imprimée sur un substrat de verre.

Encre: 50% PEG DA (Mw 700) 50% d'eau 0,0075% d'Irgacure 819 1% particules fluorescentes (diamètre 300 nm)
Distance séparant la face distale du substrat : 60µm
Pression d'injection : 100 mbar
Pression d'aspiration -150 mbar
Vitesse selon l'axe X : 0,1 mm/s
Puissance du laser : 10 mW
Résolution : 100 µm (largeur dans le plan XY)

Le test 4 est réalisé avec une distance séparant la face distale du substrat de 170 µm. Quatres lignes adjacentes sont tracées sur une longeur de 1 mm avec une largeur de 100 µm.

La hauteur de la face distale est augmentée de 100 µm pour la porter à 270 µm et deux lignes sont tracées uniquement sur le côté. (1 mm de longueur et 100 µm de largeur)

Puis, on augmente à nouveau la distance de 150 µm la portant à 420 µm et deux lignes son tracées seulement sur le côté. (1 mm de longueur et 50 µm de largeur)

## Revendications

1. Tête **(6)** d'impression microfluidique d'une imprimante **(1)** comprenant une face **(19)** distale et :
- au moins un premier canal appelé canal **(14)** d'injection configuré pour injecter une encre sur un substrat **(40),** le canal **(14)** d'injection débouchant sur la face **(19)** distale par un premier orifice appelé orifice **(20)** d'injection,
- au moins un deuxième canal appelé canal **(15)** d'aspiration configuré pour aspirer l'encre entre autres, le canal **(15)** d'aspiration débouchant sur la face **(19)** distale par un deuxième orifice appelé orifice **(21)** d'aspiration,
la tête (6) d'impression étant **caractérisée en ce qu'**elle comprend:
- une gorge **(31)** définie sur la face **(19)** distale et dans laquelle l'orifice **(21)** d'aspiration débouche,
- au moins une source **(9)** de lumière ou un guide **(17)** d'onde optique configuré(e) pour transmettre la lumière émise par une source **(9)** de lumière,
et **en ce que** la source **(9)** de lumière ou le guide **(17)** d'onde optique est configuré(e) pour projeter un faisceau lumineux sur une zone **(22)** éclairante située sur la face **(19)** distale, la zone **(22)** éclairante étant à son tour configurée pour projeter un faisceau lumineux depuis la face **(19)** distale.

2. Tête **(6)** d'impression selon la revendication 1 dans laquelle elle comprend un corps **(23)** et un embout **(24)** rapporté sur le corps **(23),** l'embout **(24)** comprenant l'orifice **(20)** d'injection, l'orifice **(21)** d'aspiration, la gorge **(31)** et la zone (22) éclairante.

3. Tête **(6)** d'impression selon la revendication 2 dans laquelle le corps **(23)** comprend le canal **(14)** d'injection, le canal **(15)** d'aspiration et la source **(9)** de lumière ou le guide **(39)** d'onde optique, respectivement situés en regard de l'orifice **(20)** d'injection, de l'orifice **(21)** d'aspiration et de la zone (22) éclairante.

4. Tête **(6)** d'impression selon l'une des revendications 2 ou 3 dans laquelle l'embout **(24)** est réalisé en matériau transparent de préférence en élastomère Poly Dimethil Siloxane.

5. Tête **(6)** d'impression selon l'une des revendications 2 à 4 dans laquelle la gorge **(31)** s'étend en profondeur selon un axe longitudinal de l'embout **(24)** et présente une forme entourant la zone **(22)** éclairante.

6. Tête **(6)** d'impression selon l'une des revendications 2 à 5 dans laquelle la gorge **(31)** s'étend sur la circonférence de l'embout **(24)** selon un secteur angulaire compris entre 180° et 340° et de préférence 320°.

7. Tête **(6)** d'impression selon l'une des revendications précédentes dans laquelle la gorge **(31)** a une largeur radiale sensiblement constante comprise entre 20 et 500 µm et de préférence 100 et 300 µm.

8. Tête d'impression selon l'une quelconque des revendications précédentes dans laquelle une profondeur axiale de la gorge **(31)** mesurée entre la face **(19)** distale et un point le plus profond situé dans la gorge **(31)** est compris entre 20 et 500 µm et de préférence 100 et 300 µm.

9. Tête **(6)** d'impression selon l'une quelconque des revendications précédentes dans laquelle la profondeur de la gorge **(31)** est sensiblement constante.

10. Tête **(6)** d'impression selon l'une des revendications précédentes dans laquelle la zone **(22)** éclairante a un diamètre compris entre cinq et cinq cent micromètres.

11. Tête **(6)** d'impression selon l'une quelconque des revendications précédentes dans laquelle l'orifice **(20)** d'injection et l'orifice **(21)** d'aspiration ont des diamètres compris entre vingt micromètres et un millimètre.

12. Tête **(6)** d'impression selon l'une des revendications précédentes dans laquelle la zone **(22)** éclairante comporte une plateforme **(54)** faisant saillie de la face **(19)** distale, sur une distance comprise entre 50 et 500 µm, de préférence 100 µm.

13. Tête **(6)** d'impression selon l'une des revendications précédentes dans laquelle l'embout **(24)** comporte une couronne **(57)** annulaire s'étendant sur le périmètre dudit embout **(24),** la couronne **(57)** faisant saillie de la face **(19)** distale.

14. Tête **(6)** d'impression selon la revendication 13 dans laquelle l'embout **(24)** comporte une cavité **(55)** avant agencée entre l'orifice **(20)** d'injection et la plateforme **(54),** et, une cavité **(56)** arrière agencée entre la plateforme **(54)** et l'orifice **(21)** d'aspiration.

15. Tête **(6)** d'impression selon l'une quelconque des revendications 1 à 7 dans laquelle la gorge **(31)** présente une profondeur axiale variable.

16. Tête **(6)** d'impression selon la revendication 13 dans laquelle, la couronne, comporte un rebord **(58)** annulaire prolongeant la couronne **(57),** ledit rebord **(58)** annulaire étant orienté en direction de la plateforme **(54)** de sorte que, le rebord **(58)** annulaire surplombe partiellement l'orifice **(21)** d'aspiration et/ou l'orifice **(20)** d'injection.

17. Imprimante **(1)** comprenant :
- au moins un réservoir **(2)** d'encre,
- au moins une pompe **(3)** d'injection apte à mettre sous pression le réservoir **(2)** d'encre,
- un répartiteur **(4)** relié de manière fluidique au réservoir **(2)** d'encre, le répartiteur **(4)** étant apte à contrôler le débit d'encre prélevé du réservoir **(2)** d'encre,
- un bras **(5)** robotisé apte à se déplacer ou un socle robotisé apte à se déplacer,
- au moins une tête **(6)** d'impression selon l'une des revendications 1 à 16 montée sur le bras **(5)** robotisé lorsque l'imprimante (1) comprend un bras **(5)** robotisé ou montée sur un support fixe de l'imprimante lorsque l'imprimante **(1)** comprend un socle robotisé, l'au moins une tête (6) d'impression étant reliée fluidiquement au répartiteur **(4)** pour alimenter celle-ci en encre, la tête **(6)** d'impression étant apte à injecter de l'encre sur un substrat **(40),**
- des moyens **(7)** d'aspiration reliés fluidiquement à la tête **(6)** d'impression pour aspirer de l'encre injectée par la tête **(6)** d'impression,
- un réservoir **(2)** d'aspiration relié fluidiquement aux moyens **(7)** d'aspiration pour stocker l'encre aspirée,
- au moins une source **(9)** de lumière apte à polymériser l'encre au niveau de la tête **(6)** d'impression dans le cas où la tête **(6)** d'impression ne comprend pas de source **(9)** de lumière,
- une unité **(10)** informatique connectée et contrôlant la pompe **(3)** d'injection, le répartiteur **(4),** le bras **(5)** robotisé ou selon le cas le socle robotisé, les moyens **(7)** d'aspiration et la source **(9)** de lumière.

18. Procédé d'impression au moyen d'une imprimante **(1)** comprenant :
- au moins un réservoir **(2)** d'encre,
- au moins une pompe **(3)** d'injection apte à mettre sous pression le réservoir **(2)** d'encre,
- un répartiteur **(4)** relié de manière fluidique au réservoir **(2)** d'encre, le répartiteur **(4)** étant apte à contrôler le débit d'encre prélevé du réservoir **(2)** d'encre,
- un bras **(5)** robotisé apte à se déplacer ou un socle robotisé apte à se déplacer,
- au moins une tête **(6)** d'impression selon l'une des revendications 1 à 16 montée sur le bras **(5)** robotisé lorsque l'imprimante **(1)** comprend un bras **(5)** robotisé ou ladite au moins une tête **(6)** dimpression est montée sur un support fixe de l'imprimante lorsque l'imprimante **(1)** comprend un socle robotisé, l'au moins une tête **(6)** d'impression étant reliée fluidiquement au répartiteur **(4)** pour alimenter ladite au moins une tête **(6)** d'impression en encre, ladite au moins une tête **(6)** d'impression étant apte à injecter de l'encre sur un substrat **(40),**
- des moyens **(7)** d'aspiration reliés fluidiquement à la tête **(6)** d'impression pour aspirer de l'encre injectée par la tête **(6)** d'impression,
- un réservoir **(2)** d'aspiration relié fluidiquement aux moyens **(7)** d'aspiration pour stocker l'encre aspirée,
- au moins une source **(9)** de lumière apte à polymériser l'encre au niveau de la tête **(6)** d'impression dans le cas où la tête **(6)** d'impression ne comprend pas de source **(9)** de lumière,
- une unité **(10)** informatique connectée et contrôlant la pompe **(3)** d'injection, le répartiteur **(4),** le bras **(5)** robotisé ou selon le cas le socle robotisé, les moyens **(7)** d'aspiration et la source **(9)** de lumière,
le procédé étant mis en œuvre au moyen d'un programme informatique implémenté dans l'unité **(10)** informatique, ce procédé comprenant :
- une étape d'injection d'au moins une encre au moyen de la tête **(6)** d'impression sur un substrat **(40)** immergé,
- une étape d'aspiration via la tête **(6)** d'impression de l'encre,
- un étape de polymérisation au moyen de la au moins une source **(9)** de lumière de l'encre injectée,
ces étapes étant réalisées simultanément pendant au moins une partie de la durée d'impression.

19. Procédé d'impression selon la revendication 18 dans lequel celui-ci comprend une étape de déplacement du bras **(5)** robotisé dans au moins une direction de l'espace.

20. Procédé d'impression selon la revendication 18 dans lequel celui-ci comprend une étape de déplacement du socle robotisé dans au moins une direction de l'espace.

## Patentansprüche

1. Mikrofluidik-Druckkopf **(6)** eines Druckers **(1),** der eine distale Fläche **(19)** umfasst, und:
- mindestens einen ersten Kanal, Einspritzkanal **(14)** genannt, der dazu konfiguriert ist, eine Tinte auf ein Substrat **(40)** einzuspritzen, wobei der Einspritzkanal **(14)** auf der distalen Fläche **(19)** durch eine erste Öffnung, Einspritzöffnung **(20)** genannt, mündet,
- mindestens einen zweiten Kanal, Ansaugkanal **(15)** genannt, der dazu konfiguriert ist, unter anderem die Tinte anzusaugen, wobei der Ansaugkanal **(15)** auf der distalen Fläche **(19)** durch eine zweite Öffnung, Ansaugöffnung **(21)** genannt, mündet, wobei der Druckkopf **(6) dadurch gekennzeichnet ist, dass** er umfasst:
- eine Nut **(31),** die auf der distalen Fläche **(19)** definiert ist, und in die die Ansaugöffnung **(21)** mündet,
- mindestens eine Lichtquelle **(9)** oder einen Lichtwellenleiter **(17),** die/der dazu konfiguriert ist, Licht, das von einer Lichtquelle **(9)** abgegeben wird, zu übertragen,
und **dass** die Lichtquelle **(9)** oder der Lichtwellenleiter **(17)** dazu konfiguriert ist, einen Lichtstrahl auf eine beleuchtende Zone **(22),** die auf der distalen Fläche **(19)** liegt, zu projizieren, wobei die beleuchtende Zone **(22)** wiederum dazu konfiguriert ist, einen Lichtstrahl von der distalen Fläche **(19)** ausgehend zu projizieren.

2. Druckkopf **(6)** nach Anspruch 1, der einen Körper **(23)** und einen Ansatz **(24)** umfasst, der auf dem Körper **(23)** angebaut ist, umfasst, wobei der Ansatz **(24)** die Einspritzöffnung **(20),** die Ansaugöffnung **(21),** die Nut **(31)** und die beleuchtende Zone **(22)** umfasst.

3. Druckkopf **(6)** nach Anspruch 2, wobei der Körper **(23)** den Einspritzkanal **(14),** den Ansaugkanal **(15)** und die Lichtquelle **(9)** oder den Lichtwellenleiter **(39)** umfasst, die jeweils der Einspritzöffnung **(20),** der Ansaugöffnung **(21)** und der beleuchtenden Zone **(22)** gegenüberliegen.

4. Druckkopf **(6)** nach einem der Ansprüche 2 oder 3, wobei der Ansatz **(24)** aus einem durchsichtigen Material, vorzugsweise aus Polydimethylsiloxanelastomer, hergestellt ist.

5. Druckkopf **(6)** nach einem der Ansprüche 2 bis 4, wobei sich die Nut **(31)** in die Tiefe entlang einer Längsachse des Ansatzes **(24)** erstreckt und eine Form aufweist, die die beleuchtende Zone **(22)** umgibt.

6. Druckkopf **(6)** nach einem der Ansprüche 2 bis 5, wobei sich die Nut **(31)** auf dem Umfang des Ansatzes **(24)** gemäß einem Winkelsektor, der zwischen 180° und 340° und vorzugsweise 320° liegt, erstreckt.

7. Druckkopf **(6)** nach einem der vorstehenden Ansprüche, bei dem die Nut **(31)** eine im Wesentlichen konstante radiale Breite aufweist, die zwischen 20 und 500 µm und vorzugsweise zwischen 100 und 300 µm liegt.

8. Druckkopf nach einem der vorstehenden Ansprüche, wobei eine axiale Tiefe der Nut **(31)** zwischen der distalen Fläche **(19)** und einem tiefsten Punkt, der in der Nut **(31)** liegt, gemessen wird, zwischen 20 und 500 µm und vorzugsweise zwischen 100 und 300 µm liegt.

9. Druckkopf **(6)** nach einem der vorstehenden Ansprüche, wobei die Tiefe der Nut **(31)** im Wesentlichen konstant ist.

10. Druckkopf **(6)** nach einem der vorstehenden Ansprüche, wobei die beleuchtende Zone **(22)** einen Durchmesser aufweist, der zwischen fünf und fünfhundert Mikrometer liegt.

11. Druckkopf **(6)** nach einem der vorstehenden Ansprüche, wobei die Einspritzöffnung **(20)** und die Ansaugöffnung **(21)** Durchmesser aufweisen, die zwischen zwanzig Mikrometer und einem Millimeter liegen.

12. Druckkopf **(6)** nach einem der vorstehenden Ansprüche, bei dem die beleuchtende Zone **(22)** eine Plattform **(54)** umfasst, die von der distalen Fläche **(19)** auf einer Distanz zwischen 50 und 500 µm, vorzugsweise 100 µm, vorragt.

13. Druckkopf **(6)** nach einem der vorstehenden Ansprüche, wobei der Ansatz **(24)** einen Ringkranz **(57)** umfasst, der sich auf dem Umfang des Ansatzes **(24)** erstreckt, wobei der Ringkranz **(57)** von der distalen Fläche **(19)** vorragt.

14. Druckkopf **(6)** nach Anspruch 13, wobei der Ansatz **(24)** einen vorderen Hohlraum **(55)** aufweist, der zwischen der Einspritzöffnung **(20)** und der Plattform **(54)** eingerichtet ist, und einen hinteren Hohlraum **(56),** der zwischen der Plattform **(54)** und der Ansaugöffnung **(21)** eingerichtet ist.

15. Druckkopf **(6)** nach einem der Ansprüche 1 bis 7, wobei die Nut **(31)** eine variable axiale Tiefe aufweist.

16. Druckkopf **(6)** nach Anspruch 13, wobei der Kranz eine Ringrandleiste **(58)** umfasst, die den Kranz **(57)** verlängert, wobei die Ringrandleiste **(58)** in Richtung der Plattform **(54)** derart orientiert ist, dass die Ringrandleiste **(58)** teilweise über die Ansaugöffnung **(21)** und/oder die Einspritzöffnung **(20)** auskragt.

17. Drucker **(1),** der umfasst:
- mindestens einen Tintenbehälter **(2),**
- mindestens eine Einspritzpumpe **(3),** die dazu geeignet ist, den Tintenbehälter **(2)** mit Druck zu beaufschlagen,
- einen Verteiler **(4),** der fluidisch mit dem Tintenbehälter **(2)** verbunden ist, wobei der Verteiler **(4)** dazu geeignet ist, den Tintendurchsatz, der aus dem Tintenbehälter **(2)** entnommen wird, zu steuern,
- einen Roboterarm **(5),** der dazu geeignet ist, sich zu verlagern, oder einen Robotersockel, der dazu geeignet ist, sich zu verlagern,
- mindestens einen Druckkopf **(6)** nach einem der Ansprüche 1 bis 16, der auf dem Roboterarm **(5)** montiert ist, wenn der Drucker **(1)** einen Roboterarm **(5)** umfasst, oder auf einem fixen Träger des Druckers montiert ist, wenn der Drucker **(1)** einen Robotersockel umfasst, wobei der mindestens eine Druckkopf **(6)** fluidisch mit dem Verteiler **(4)** verbunden ist, um diesen mit Tinte zu versorgen, wobei der Druckkopf **(6)** dazu geeignet ist, Tinte auf ein Substrat **(40)** einzuspritzen,
- Ansaugmittel **(7),** die fluidisch mit dem Druckkopf **(6)** verbunden sind, um die von dem Druckkopf **(6)** eingespritzte Tinte anzusaugen,
- einen Ansaugbehälter **(2),** der fluidisch mit den Ansaugmitteln **(7)** verbunden ist, um die angesaugte Tinte zu lagern,
- mindestens eine Lichtquelle **(9),** die dazu geeignet ist, die Tinte im Bereich des Druckkopfs **(6)** in dem Fall zu polymerisieren, dass der Druckkopf **(6)** keine Lichtquelle **(9)** umfasst,
- eine Recheneinheit **(10),** die mit der Einspritzpumpe **(3),** dem Verteiler **(4),** dem Roboterarm **(5)** oder, je nach Fall, dem Robotersockel, den Ansaugmitteln **(7)** und der Lichtquelle **(9)** verbunden ist und diese steuert.

18. Druckverfahren mittels eines Druckers **(1),** der umfasst:
- mindestens einen Tintenbehälter **(2),**
- mindestens eine Einspritzpumpe **(3),** die dazu geeignet ist, den Tintenbehälter **(2)** mit Druck zu beaufschlagen,
- einen Verteiler **(4),** der fluidisch mit dem Tintenbehälter **(2)** verbunden ist, wobei der Verteiler **(4)** dazu geeignet ist, den Tintendurchsatz, der aus dem Tintenbehälter **(2)** entnommen wird, zu steuern,
- einen Roboterarm **(5),** der dazu geeignet ist, sich zu verlagern, oder einen Robotersockel, der dazu geeignet ist, sich zu verlagern,
- mindestens einen Druckkopf **(6)** nach einem der Ansprüche 1 bis 16, der auf dem Roboterarm **(5)** montiert ist, wenn der Drucker **(1)** einen Roboterarm **(5)** umfasst, oder wobei der mindestens eine Druckkopf **(6)** auf einem fixen Träger des Druckers montiert ist, wenn der Drucker **(1)** einen Robotersockel umfasst, wobei der mindestens eine Druckkopf **(6)** fluidisch mit dem Verteiler **(4)** verbunden ist, um den mindestens einen Druckkopf **(6)** mit Tinte zu versorgen, wobei der mindestens eine Druckkopf **(6)** dazu geeignet ist, die Tinte auf ein Substrat **(40)** einzuspritzen,
- Ansaugmittel **(7),** die fluidisch mit dem Druckkopf **(6)** verbunden sind, um Tinte, die von dem Druckkopf **(6)** eingespritzt wird, anzusaugen,
- einen Ansaugbehälter **(2),** der fluidisch mit den Ansaugmitteln **(7)** verbunden ist, um die angesaugte Tinte zu lagern,
- mindestens eine Lichtquelle **(9),** die dazu geeignet ist, die Tinte im Bereich des Druckkopfs **(6)** in dem Fall zu polymerisieren, dass der Druckkopf **(6)** keine Lichtquelle **(9)** umfasst,
- eine Recheneinheit **(10),** die mit der Einspritzpumpe **(3),** dem Verteiler **(4),** dem Roboterarm **(5)** oder, je nach Fall, mit dem Robotersockel, den Ansaugmitteln **(7)** und der Lichtquelle **(9)** verbunden ist und diese steuert,
wobei das Verfahren mittels eines Rechenprogramms umgesetzt wird, das in der Recheneinheit **(10)** implementiert ist, wobei dieses Verfahren umfasst:
- einen Schritt zum Einspritzen mindestens einer Tinte mittels des Druckkopfs **(6)** auf ein eingetauchtes Substrat **(40),**
- einen Schritt zum Ansaugen der Tinte über den Druckkopf **(6),**
- einen Schritt zum Polymerisieren der eingespritzten Tinte mittels der mindestens einen Lichtquelle **(9),**
wobei diese Schritte gleichzeitig während mindestens eines Teils der Druckzeit ausgeführt werden.

19. Druckverfahren nach Anspruch 18, wobei dieses einen Verlagerungsschritt des Roboterarms **(5)** in mindestens eine räumliche Richtung umfasst.

20. Druckverfahren nach Anspruch 18, wobei dieses einen Verlagerungsschritt des Robotersockels in mindestens eine räumliche Richtung umfasst.

## Claims

1. Microfluidic print head (6) of a printer (1), comprising a distal face (19) and:
- at least one first channel referred to as injection channel (14) configured for injecting an ink onto a substrate (40), the injection channel (14) leading onto the distal face (19) through a first opening referred to as injection opening (20),
- at least one second channel referred to as suction channel (15) configured for suctioning up the ink, among others, the suction channel (15) leading onto the distal face (19) through a second opening referred to as suction opening (21),
the print head (6) being **characterised in that** it comprises:
- a groove (31) defined on the distal face (19) and wherein the suction opening (21) leads,
- at least one light source (9) or optical waveguide (17) configured for transmitting the light emitted by a light source (9),
and **in that** the light source (9) or optical waveguide (17) is configured to project a beam of light onto a lighting zone (22) located on the distal face (19), the lighting zone (22) in turn being configured to project a beam of light from the distal face (19) thereof.

2. Print head (6) according to claim 1, wherein it comprises a body (23) and an end-piece (24) provided on the body (23), the end-piece (24) comprising the injection opening (20), the suction opening (21), the groove (31) and the lighting zone (22).

3. Print head (6) according to claim 2, wherein the body (23) comprises the injection channel (14), the suction channel (15) and the light source (9) or the optical waveguide (39), respectively located opposite the injection opening (20), the suction opening (21) and the lighting zone (22);

4. Print head (6) according to one of claims 2 or 3, wherein the end-piece (24) is made of a transparent material, preferably of a Polydimethylsiloxane elastomer.

5. Print head (6) according to one of claims 2 to 4, wherein the groove (31) extends in depth along a longitudinal axis of the end-piece (24), and has a shape surrounding the lighting zone (22).

6. Print head (6) according to one of claims 2 to 5, wherein the groove (31) extends over the circumference of the end-piece (24) according to an angular sector ranging from 180° to 340°, and preferably 320°.

7. Print head (6) according to one of the preceding claims, wherein the groove (31) has a radial width that is substantially constant and ranges from 20 to 500µm, and preferably from 100 to 300µm.

8. Print head according to any one of the preceding claims, wherein an axial depth of the groove (31) measured between the distal face (19) and a deeper point located in the groove (31) ranges from 20 to 500µm and preferably from 100 to 300µm.

9. Print head (6) according to any one of the preceding claims, wherein the depth of the groove (31) is substantially constant.

10. Print head (6) according to one of the preceding claims, wherein the lighting zone (22) has a diameter ranging from five to five hundred micrometres.

11. Print head (6) according to any one of the preceding claims, wherein the injection opening (20) and the suction opening (21) have diameters ranging from twenty micrometres and one millimetre.

12. Print head (6) according to one of the preceding claims, wherein the lighting zone (22) comprises a platform (54) protruding from the distal face (19), by a distance ranging from 50 to 500µm, preferably 100µm.

13. Print head (6) according to one of the preceding claims, wherein the end-piece (24) comprises an annular crown (57) extending over the perimeter of said end-piece (24), the crown protruding from the distal face (19).

14. Print head (6) according to claim 13, wherein the end-piece (24) comprises a front cavity (55) arranged between the injection opening (20) and the platform (54), and a rear cavity (56) arranged between the platform (54) and the suction opening (21).

15. Print head (6) according to any one of claims 1 to 7, wherein the groove (31) has a variable axial depth.

16. Print head (6) according to claim 13, wherein the crown comprises an annular edge (58) extending the crown (57), said annular edge being oriented in the direction of the platform (54) such that the annular edge (58) partially overlaps with the suction opening (21) and/or the injection opening (20).

17. Printer (1) comprising:
- at least one ink reservoir (2),
- at least one injection pump (3) able to pressurise the ink reservoir (2),
- a distributor (4) fluidly connected to the ink reservoir (2), the distributor (4) being able to control the flow rate of the ink collected from the ink reservoir (2),
- a robotic arm (5) able to move or a robotic base able to move,
- at least one print head (6) according to one of claims 1 to 16 mounted on the robotic arm (5) when the printer (1) comprises a robotic arm (5), or on a fixed support of the printer when the printer (1) comprises a robotic base, the at least one print head (6) being fluidly connected to the distributor (4) to supply it with ink, the at least one print head (6) being able to inject ink onto a substrate (40),
- suction means (7) fluidly connected to the print head (6) in order to suck the ink injected by the print head (6),
- a suction reservoir (2) fluidly connected to the suction means (7) to store the suctioned ink,
- at least one light source (9) able to polymerise the ink at the level of the print head (6) if the print head does not comprise a light source (9),
- a connected computer unit (10) that controls the injection pump (3), the distributor (4), the robotic arm (5) or, depending on the case, the robotic base, the suction means (7) and the light source (9).

18. Method for printing with a printer (1) comprising:
- at least one ink reservoir (2),
- at least one injection pump (3) able to pressurise the ink reservoir (2),
- a distributor (4) fluidly connected to the ink reservoir (2), the distributor (4) being able to control the flow rate of the ink collected from the ink reservoir (2),
- a robotic arm (5) able to move or a robotic base able to move,
- at least one print head (6) according to one of claims 1 to 16, mounted on the robotic arm (5) when the printer (1) comprises a robotic arm (5), or said at least one print head (6) mounted on a fixed support of the printer when the printer (1) comprises a robotic base, the at least one print head (6) being fluidly connected to the distributor (4) to supply ink to the at least one print head (6), the at least one print head being able to inject ink onto a substrate (40),
- suction means (7) fluidly connected to the print head (6) in order to suck the ink injected by the print head (6),
- a suction reservoir (2) fluidly connected to the suction means (7) to store the suctioned ink,
- at least one light source (9) able to polymerise the ink at the level of the print head (6) if the print head does not comprise a light source (9),
- a connected computer unit (10) that controls the injection pump (3), the distributor (4), the robotic arm (5) or, depending on the case, the robotic base, the suction means (7) and the light source (9),
said method being implemented by means of a computer program in the computer unit (10), the method comprising:
- a step of injecting at least one ink by means of the print head (6) on an immersed substrate (40),
- a step of suctioning the ink through the print head (6),
- a step of polymerising the injected ink by means of the at least one light source (9),
these steps being performed simultaneously during at least some of the printing time.

19. Printing method according to claim 18, wherein it comprises a step of moving the robotic arm (5) in at least one spatial direction.

20. Printing method according to claim 18, wherein it comprises a step of moving the robotic base in at least one spatial direction.
